## Europäisches Patentamt

## European Patent Office

## Office européen des brevets

(19)

(11) Numéro de publication: **0 022 722 B1**

(12) **FASCICULE DE BREVET EUROPÉEN**

(45) Date de publication du fascicule du brevet:
14.01.87

(51) Int. Cl.⁴: **A 61 B 6/02, H 05 G 1/62**

(21) Numéro de dépôt: **80401048.6**

(22) Date de dépôt: **11.07.80**

(54) **Appareil comportant un tomodensitomètre et procédé d'utilisation de cet appareil en cinédensigraphie.**

(30) Priorité: **17.07.79 FR 7918463**

(43) Date de publication de la demande:
**21.01.81 Bulletin 81/3**

(45) Mention de la délivrance du brevet:
**14.01.87 Bulletin 87/3**

(84) Etats contractants désignés:
**DE GB SE**

(56) Documents cité:
**FR-A-2 020 416**
**FR-A-2 073 858**
**FR-A-2 304 320**
**FR-A-2 345 983**
**FR-A-2 385 375**
**FR-A-2 403 059**

**IEEE TRANSACTIONS ON NUCLEAR SCIENCE (US), vol. NS-26, no. 2, part II, avril 1979, New York, US R.A. ROBB et al. "The DSR: A high-speed three-dimensional X-ray computed tomography system for dynamic spatial reconstruction of the heart and circulation", pages 2713-2717.**
**IEEE TRANSACTIONS ON NUCLEAR SCIENCE (US), vol. NS-26, no. 2, Part II, avril 1979, New York, US D.P. BOYD: "A proposed dynamic cardiac 3-D densitometer for early detection and evaluation of heart disease", pages 2724-2727**

(73) Titulaire: **THOMSON- CSF, 173, Boulevard Haussmann, F-75379 Paris Cedex 08 (FR)**

(72) Inventeur: **Klausz, Rémy, "THOMSON- CSF" SCPI 173, Bld Haussmann, F-75360 Paris Cedex 08 (FR)**

(74) Mandataire: **Chaverneff, Vladimir, THOMSON- CSF SCPI 19, avenue de Messine, F-75008 Paris (FR)**

## Description

La présente invention concerne un appareil comportant un tomodensitomètre et utilisé en cinédensigraphie et un procédé d'utilisation de cet appareil.

On connaît des appareils de tomographie d'après les documents FR-A-2 304 320; IEEE Transactions on Nuclear Science, Vol. NS-26, N° 2, Part II, pages 2713 à 2717; FR-A-2 385 375 et FR-A-2 345 983. Aucun de ces documents ne suggère l'utilisation de tels appareils en cinédensigraphie.

Les techniques de cinédensigraphie sont connues en elles-mêmes depuis longtemps. Elles ont essentiellement été développées en France par M. Henri-Maurice MARCHAL et sont décrites par exemple au tome 1 de l'ouvrage intitulé "Traité de radiodiagnostic" de J. DUTREIX, V. BISMUTH et M. LAVAL-JEANTET publié aux éditions MASSON et Cie, page 372 et suivantes.

La méthode connue jusqu'a présent pour obtenir un enregistrement cinédensigraphique consistait à irradier par un faisceau de rayons X la zone du patient à observer, à recueillir sur une cellule de détection les rayons X transmis, à les convertir en un signal électrique et à enregistrer l'amplitude de ce signal en fonction du temps.

Cette méthode étant principalement destinée à repérer et localiser des anomalies dans les poumons. En effet, une anomalie telle qu'une tumeur modifie les mouvements du poumon lui-même dans sa fonction de respiration et affecte le réseau vasculaire. Si l'on peut observer l'amplitude des mouvements dans les différentes zones des poumons, on peut repérer les zones dans lesquelles ces mouvements sont anormalement faibles et correspondent a une anomalie. Il est également intéressant de pouvoir observer comment se comporte dans le temps la densité de différentes zones observées, même en l'absence de déformations d'organes.

Dans cette méthode connue jusqu'alors, le signal électrique recueilli, qui varie dans le temps, correspond à une intégration de l'information sur toute la zone vue par la cellule de détection; cette intégration a lieu non seulement sur l'épaisseur du sujet traversée par le faisceau de rayons X, mais encore sur la surface du sujet correspondant à la section droite du faisceau qui l'irradie.

Le fait qu'il était nécessaire de disposer d'un équipement spécialisé a fait que cette technique de la cinédensigraphie est pratiquement toujours restée au stade du laboratoire malgré les résultats intéressants qu'elle pouvait procurer sur le comportement dans le temps d'organes soumis aux rayons X.

Par ailleurs, on connaît bien maintenant les techniques de la tomodensitométrie, ou tomographie axiale transverse. Par opposition à la cinédensigraphie ou l'on cherchait à connaître les variations d'atténuation en une petite zone en fonction du temps, la tomodensitométrie qui permet de reconstituer la valeur du coefficient d'atténuation linéaire en tout point d'une coupe ou section transverse et de reconstruire l'image de cette section, ne peut donner de bons résultats que si l'objet examiné est rigoureusement identique à lui-même à tout instant de l'exploration. Faute de cette absence de variation dans le temps de la forme et/ou de la densité de l'objet examiné, l'image reconstituée est inexacte ou brouillée.

La présente invention concerne un nouveau appareil comportant un tomodensitomètre et un procédé d'utilisation d'un tel appareil qui permet, tout en lui laissant ses possibilités de fonctionnement normal en tomographie axiale transverse, de l'utiliser pour faire de la cinédensigraphie.

Ainsi quelques adaptations peu coûteuses apportées à un tomodensitomètre classique permettent à un centre d'examen médical qui dispose de cet appareil de la faire fonctionner selon le procédé de la présente invention et de réaliser des examens de cinédensigraphie.

Il est très important de noter que ces adaptations n'empêchent nullement de faire fonctionner l'appareil en mode tomodensitométrique normal, une simple commutation permettant de passer d'une technique à l'autre. La combinaison des deux techniques est d'ailleurs très avantageuse comme on le verra dans la description donnée ci-après.

Selon l'invention, un appareil comportant un tomodensitomètre du type comprenant notamment un dispositif de mesure d'absorption de rayons X dans un sujet, ce dispositif de mesure incluant une source de rayons X tournante émettant un faisceau en forme d'éventail plat et au moins N détecteurs placés en vis-à-vis de ladite source pour sélectionner N faisceaux élémentaires dans ledit faisceau, N étant un nombre supérieur ou égal à un, des moyens de traitement et de mémorisation connectés pour recevoir les signaux délivrés par les N détecteurs et des moyens de visualisation, la sortie desdits moyens de traitement et de mémorisation étant reliée auxdits moyens de visualisation, est caractérisé en ce qu'il comporte en outre des moyens de commande d'actionnement desdits moyens de traitement et de mémorisation, formant horloge, mis en oeuvre spécifiquement pour stocker dans lesdits moyens de mémorisation des informations de cinédensigraphie lorsque ladite source de rayons X est immobilisée et émet dans une position donnée par rapport à une section choisie dudit sujet et en vis-à-vis de N détecteurs précités, les moyens formant horloge étant utilisés, en mode cinédensigraphie, à la place des moyens de repérage qui, dans le mode tomodensitométrie, indiquent la position angulaire du dispositif de mesure.

Selon l'invention, un procédé d'utilisation dun tel appareil pour obtenir, conformément à la technique de la cinédensigraphie, des informations caractéristiques de l'evolution dans le temps de la densité de parties dun sujet irradié par des rayons X délivrés par ledit appareil, est caractérisé en ce qu'il consiste:

- à maintenir ladite source de rayons X dans une position donnée par rapport à une section choisie dudit sujet et en vis-à-vis de N détecteurs précités;

- à soumettre ladite section aux rayons X délivrés par la source;

- à commander l'actionnement desdits moyens de traitement et de mémorisation au rythme des moyens formant horloge, spécifique au mode opératoire de cinédensigraphie; et

- à visualiser des informations disponibles à la sortie desdits moyens de mémorisation et de traitement sans les soumettres à des algorithmes de reconstruction par tomodensitométrie, ces informations étant représentatives de l'amplitude des successions de N signaux résultant de la lecture des N détecteurs en fonction du temps pour un ou plusieurs faisceaux élémentaires donnés et/ou en même temps pour les différents faisceaux élémentaires.

La présente invention sera mieux comprise à la lecture de la description suivante donnée à titre d'exemple non limitatif et illustrée par les figures annexées qui représentent:

- la figure 1, une vue très schématique des éléments essentiels dun exemple de réalisation dun tomodensitomètre utilisé selon le procédé de l'invention;

- les figures 2,3 et 4, des exemples de modes de strockage et de visualisation des informations élaborées par l'appareil de la figure 1;

- la figure 5 (a) et (b), des vues très schématiques d'un mode d'utilisation particulier de l'appareil de l'invention.

Il est à noter que le tomodensitomètre plus particulièrement utilisé ici a un dispositif de mesure d'absorption des rayons X à faisceau en éventail et à N détecteurs. Ce peut être par exemple un tomodensitomètre à un détecteur unique déplacé en translation dans le plan de la section à observer de manière que les N positions successivement prises par l'ensemble source-détecteur pendant le temps $\Delta$ t d'élaboration des N signaux donnent les N faisceaux élémentaires qui engendrent ces N signaux. Dans un tel cas, le temps d'élaboration des N signaux correspondant à un échantillonnage n'est pas négligeable; c'est pourquoi les versions à N détecteurs opérant simultanément sont préférables pour la mise en oeuvre du procédé de l'invention. Un tel tomodensitomètre à un seul détecteur peut toutefois être utilisé de façon intéressante si le temps $\Delta$ t d'élaboration des N signaux est suffisamment court par rapport à la vitesse de variation de forme et/ou de densité des organes à observer. On peut également utiliser ce type d'appareil lorsqu'on ne s'intéresse qu'à une zone très limitée du corps, l'ensemble source-détecteurs occupant alors une position fixe de façon que le pinceau de rayons X traverse cette zone, le nombre N de signaux étant alors réduit à un.

Ce peut être encore un tomodensitomètre à une source et plusieurs détecteurs opérant normalement en translation rotation lorsqu'il s'agit de faire de la tomographie axiale transverse, l'élaboration des N signaux pouvant se faire en translatant l'ensemble source-détecteurs, ou en une seule position si on ne désire pas une grande finesse d'analyse de la projection examinée en cinédensigraphie.

Ce peut enfin être un tomodensitomètre à une source et de nombreux ( > N) détecteurs disposés en couronne autour de la source. Dans ce cas N seulement des détecteurs de la couronne seront utilisés pour l'élaboration des N signaux. D'autre part lorsqu'on utilise l'un des trois types de tomodensitomètre à faisceau en éventail, on peut n'utiliser pour la cinédensigraphie qu'une partie des détecteurs atteints par le faisceau de rayonnement.

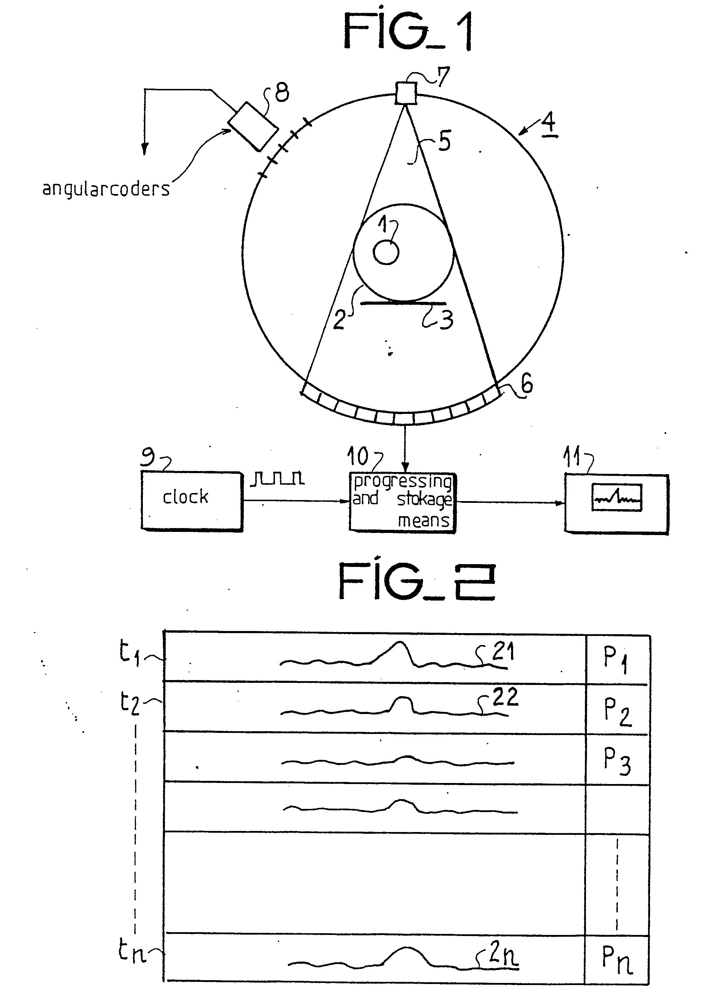

La figure 1 représente très schématiquement les éléments essentiels d'un appareil comprenant tomodensitomètre, utilisé, conformément au procédé de l'invention, pour obtenir des informations de cinédensigraphie; il permet d'observer l'évolution de la forme et/ou de la densité d'organes dans le temps, et par exemple les déformations du coeur. 1 d'un patient 2 placé sur un support 3.

Cet appareil comporte un dispositif de mesure de l'absorption des rayons X qui est celui d'un tomodensitomètre à faisceau 5 en éventail et à N détecteurs 6 disposés en arc de cercle en vis-à-vis d'une source des rayons X 7. Ce dispositif est classiquement fixé sur un statif non représenté. Les N détecteurs 6 qui recueillent le faisceau de rayons X après sa traversée de la section du patient 2 à observer déterminent N faisceaux élémentaires, constituant ici N fractions justaposées du faisceau 5, et délivrent les N signaux correspondant aux moyens de traitement et de mémorisation 10 du tomodensitomètre.

Dans l'utilisation classique d'un tomodensitomètre le dispositif de mesure (7, 6) tourne autour du patient sur au moins un demi-tour. Chaque position angulaire, déterminée par exemple au moyen de codeurs optiques tels que 8, conduit à l'élaboration de N valeurs traitées et mises en mémoire en 10; la combinaison de tous les ensembles de N valeurs c'est-à-dire de toutes les projections, par des algorithmes déterminés permet de reconstruire point par point l'image de la section examinée.

Au contraire, dans le procédé de l'invention, une fois qu'a été choisie l'orientation du dispositif de mesure (7, 6) dans le plan de la section à examiner, ce dispositif reste fixe de manière à pouvoir prélever les différents échantillons (groupes de N signaux) dans le temps d'une même projection.

Pour cela, les moyens de repérage de la position angulaire (capteurs 8 par exemple) ne commandent plus la prise de mesure. Leur action est remplacée par celle d'un générateur d'impulsions, ou horloge 9, commandant l'élaboration, le traitement et la mémorisation en

des groupes de N signaux, au rythme de cette fréquence d'horloge.

La fréquence de ces impulsions qui détermine les instants d'échantillonnage du phénomène à observer est soit constante, soit réglable par l'opérateur en fonction de la vitesse de déformation de l'organe à observer. Elle peut même être asservie à un phénomène biologique tel que l'activité électrique du coeur (électrocardiogramme ou ECG). Elle doit, dans tous les cas, être compatible avec les signaux venant, en utilisation normale du tomodensitomètre, des codeurs angulaires (tels que 8) puisque dans le procédé de l'invention le signal d'horloge simule les signaux venant de ces codeurs.

Il est à noter que l'on peut mettre à profit la possibilité qu'a le dispositif de mesure (7, 6) de tourner autour du patient pour choisir facilement la meilleure incidence pour l'observation du phénomène (déformation d'organes, variations de densité) à surveiller. Cette sélection de la meilleure incidence permettra notamment d'éviter que des parties du sujet autres que celle étudiée fournissent un signal parasite qui se superpose à celui de la partie que l'on veut observer. Cette sélection qui revient à choisir, pour les observations cinédensigraphiques, une projection prédéterminée parmi les nombreuses projections de la tomographie axiale transverse se fait en tournant l'ensemble émetteur-récepteur dans la direction angulaire voulue. On peut utiliser avantageusement, pour sélectionner cette direction, les indications fournies par une image tomodensitométrique obtenue préalablement de la section transversale correspondante. Dans le cas de l'utilisation d'un tomodensitométre à détecteurs en couronne fixe, elle se fait en tournant la seule source de rayons X dans la direction voulue et en recueillant sur les N détecteurs en vis-à-vis les informations correspondantes.

Ainsi pour faire de la cinédensigraphie avec appareil comportant un tomodensitomètre selon le procédé qui vient d'être décrit, il suffit d'utiliser une horloge 9 commandant, à la place des moyens de repérage de la position angulaire 8, la prise de données par les moyens de traitement et de mémorisation, de ne pas utiliser les algorithmes de reconstruction d'image et d'utiliser les moyens de visualisation 11 comme il va être décrit. Le passage d'un mode de fonctionnement à l'autre peut se faire par des commutations à la portée de l'homme de l'art.

Les successions de N informations ainsi obtenues correspondent à un échantillonnage dans l'espace (le long des N faisceaux élémentaires définis par les N détecteurs) et dans le temps (au rythme de l'horloge) des différents états de la section observée, et par exemple du coeur.

Il peut être intéressant, pour connaître les instants d'échantillonnage d'enregistrer simultanément à la prise des données un (ou plusieurs) signal de référence tel que le signal d'horloge ou un signal pro venant d'un capteur biologique.

Les informations ainsi élaborées à partir des successions de N signaux obtenus à chaque échantillonnage vont pouvoir être visualisées sur des moyens de visualisation 11 après d'éventuels calculs classiques en eux-mêmes tels que logarithmes, mise à l'échelle, calage par rapport à une référence... Les N informations correspondant à chaque instant d'échantillonnage définissant une projection, au lieu d'être utilisées dans le calcul de reconstruction habituel à la tomodensitométrie, sont placées dans une ligne du fichier de la mémoire destiné à contenir l'image reconstruite. Elles suivent alors le trajet normal que subirait une image reconstituée de tomodensitométrie et sont envoyées vers les moyens de stockage et de visualisation. Les courbes d'atténuation visualisant ces informations sont simples à tracer au moyen de fonctions de diagnostic sur image dont est muni le dispositif de visualisation d'un tomodensitomètre, telles que tracé de profil de densité.

Dans le cas où chaque ligne du fichier mémoire contient les N informations d'une projection, un profil suivant une ligne représentera les valeurs de la projection en un instant donné, c'est-à-dire les valeurs des N informations obtenues au cours de cet échantillonnage, un profil suivant une colonne représentera les valeurs en un point de la projection en fonction du temps, c'est-à-dire les valeurs d'un des N signaux obtenus à partir de l'un des N détecteurs.

La figure 2 représente schématiquement une telle organisation de fichier numérique où les courbes 21, 22, ... 2 n représentent chacune une projection à un instant d'échantillonnage donné $t_1, t_2 -- t_n$.

Si on a enregistré dans chaque ligne la valeur d'un (ou plusieurs) paramètre biologique symbolisé ici par les valeurs $P_1, P_2 ... P_n$, un profil de la colonne contenant ces valeurs reconstituera le tracé de la variation de ce paramètre en fonction du temps comme le ferait un enregistreur ou un oscilloscope; ce paramètre peut être par exemple l'activité électrique du coeur, ce profil particulier fournissant l'électrocardiogramme (ECG). Il est à noter que cette possibilité peut être appliquée aux images habituelles de la tomodensitométrie, en ajoutant un (ou plusieurs) canal spécial d'acquisition et numérisation de paramètres biologiques, dont les caractéristiques seront compatibles avec celles en usage sur les enregistreurs spécialisés, l'échantillonnage étant défini par l'échantillonnage angulaire et la valeur de ce paramètre étant placée par exemple en première ou dernière colonne de l'image calculée.

Il est alors possible, à partir d'un fichier ainsi constitué, de réaliser de nombreux types et combinaisons de visualisation dont quelques exemples sont donnés ci-après.

On peut visualiser, à l'image du contenu du fichier illustré par la figure 2, quelques unes des n courbes 21, 22 ... 2 n, correspondant à des instants d'échantillonnage choisis par exemple en s'aidant de la valeur à ces instants du paramètre supplémentaire; les déformations dans le temps en chaque point de la projection sont visibles sur une droite verticale.

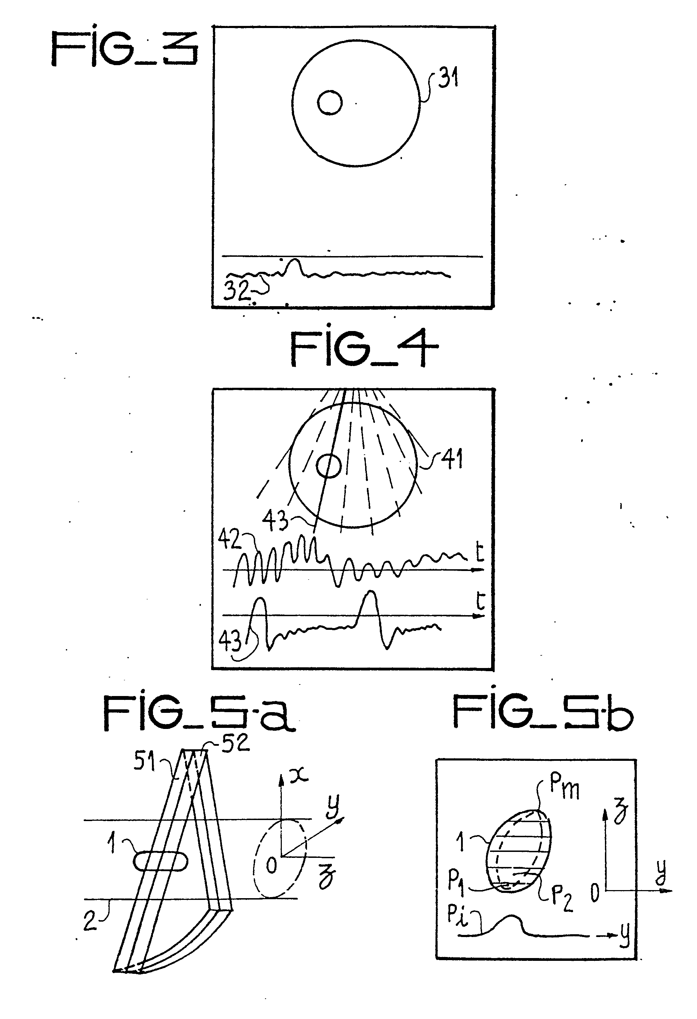

On peut combiner, comme illustré sur la figure 3, une image tomodensitométrique classique 31 de la section examinée et le profil 32, à un instant donné, de la projection suivant la direction, ou direction moyenne choisie, de cette section obtenue selon l'invention. Il est alors possible, en représentant successivement en 32 les différents profils dans le temps de cette projection de "voir bouer" cette projection. Cette dernière possibilité peut d'ailleurs être utilisée seule, sans être associée à l'image 31.

On peut, comme schématiquement représenté figure 4, associer à l'image tomodensitométrique 41 la courbe 42 de variation dans le temps en l'un quelconque des points de la projection (un des N détecteurs); le point de la projection où l'on désire ainsi voir les variations dans le temps peut être choisi en utilisant les moyens présents sur les dispositifs de visualisation des tomodensitomètres. On peut par exemple visualiser sur l'image 41 la droite 43 qui correspond au point de la projection que l'on veut observer, cette droite étant choisie parmi les N possibles par un bouton de la console de visualisation à la disposition de l'opérateur.

Il est souvent intéressant de comparer l'évolution dans le temps 42 de ce point à la variation dans le temps d'un autre paramètre biologique tel que l'ECG. On peut très facilement visualiser, sous la courbe 42, la courbe 43 représentant ce paramètre en faisant se correspondre le temps sur les deux courbes.

Ainsi le procédé de cinédensigraphie qui vient d'être décrit, outre le fait qu'il ne nécessite pas un matériel spécial puisqu'il suffit d'adapter comme il vient d'être décrit un tomodensitomètre, présente une grande souplesse dans son utilisation puisqu'il permet de représenter les variations d'un organe en fonction de l'espace (figures 2 et 3) et/ou en fonction du temps (figure 4).

Un autre mode d'utilisation de l'appareil de l'invention peut encore être très intéressant. La figure 5 permet de le comprendre.

Les mesures de cinédensigraphie sont réalisées comme précédemment, à statif fixe dans le plan de la section à observer. Par contre l'opération est faite plusieurs fois pour différentes positions relatives du statif le long de l'axe longitudinal du sujet. C'est ce que représente schématiquement la figure 5(a) où l'on voit deux positions successives 51 et 52 du faisceau en éventail décalées le long de l'axe longitudinal Oz du sujet.

Plusieurs séries de mesures vont ainsi être prises, le statif restant fixe par rapport aux axes Ox et Oy qui définissent les plans parallèles des sections du sujet, et se déplaçant le long de Oz de façon relative par rapport au sujet 2.

Il est à noter que ce mode d'utilisation se rapproche, dans un domaine différent puisqu'il s'agit ici de faire de la cinédensigraphie, de l'utilisation d'un tomodensitomètre en mode "radiographie" utilisé par de nombreux constructeurs et décrit par exemple dans la demande de brevet français de Siemens n° 77.04724 publiée sous le N° 2.345.983. Dans ce mode radiographie, le sujet est translaté parallèlement à Oz, la prise de données se faisant, comme ici, à incidence fixe. L'image qui en résulte sera équivalente à une image de radiographie conventionnelle, c'est-à-dire que le coeur I par exemple, sera représenté comme indiqué figure 5(b) en projection sur un plan (plan Oy, Oz). Cette image 1 de la figure 5(b) est constituée par la juxtaposition des différentes projections $P_1$, $P_2$ ... $P_m$ obtenues lors des m positions successives du sujet le long de Oz. On a représenté en $P_i$ sur cette même figure 5b le profil d'une de ces projections.

Les mesures cinédensigraphiques obtenues comme précédemment décrit, combinées à ce déplacement longitudinal relatif du statif par rapport au sujet vont permettre de "voir bouger" l'organe examiné, et notamment de "voir battre" le coeur 1.

Pour cela, il suffit d'effectuer pour chaque position relative du sujet par rapport au statif une série de projections en fonction du temps, au cours d'une période du phénomène à observer, battement du coeur ici.

Il est encore possible, au lieu de réaliser ainsi toutes les n projections d'une même position (51, 52, ...) consécutivement avant de changer de position, de réaliser successivement une projection par position et ceci sur un temps total court, par rapport aux variations du phénomène observé, et de recommencer, après un bref délai une telle série de mesures, autant de fois qu'il est nécessaire d'avoir d'échantillons. Il n'est alors plus nécessaire que le phénomène soit périodique. On aura finalement dans un cas comme dans l'autre, pour chaque position, n profils d'une même projection pris aux différents instants d'échantillonnage soit consécutivement, soit de façon imbriquée entre les différentes positions.

Il suffit alors de commuter successivement sur l'image visualisée, figure 5 (b), les projections $P_1$, $P_2$ .. $P_m$ correspondant aux instants d'échantillonnage pour "voir bouger" l'organe et plus particulièrement "voir battre" le coeur.

Ce mode d'utilisation de l'appareil de l'invention permet d'obtenir un résultat qui s'apparente aux résultats que donne, par des moyens très différents, la méthode de kymographie.

**Revendications**

1. Appareil comportant un tomodensitomètre du type comprenant notamment un dispositif de mesure d'absorption de rayons X dans un sujet, ce dispositif de mesure (4) incluant une source de rayons X tournante émettant un faisceau en forme déventail plat et au moins N détecteurs placés en vis-à-vis de ladite source pour sélectionner N faisceaux élémentaires dans ledit faisceau, N étant un nombre supérieur ou égal à un, des moyens de traitements et de mémorisation (10) connectés pour recevoir les signaux délivrés par les N détecteurs et des moyens de visualisation (11), la sortie desdits moyens de traitement et de mémorisation étant reliée auxdits moyens de visualisation (11), caractérisé en ce qu'il comporte en outre des moyens de commande d'actionnement (9) desdits moyens de traitement et de mémorisation, formant horloge, mis en oeuvre spécifiquement pour stocker dans lesdits moyens de mémorisation des informations de cinédensigraphie lorsque ladite source de rayons X est immobilisée et émet dans une position donnée par rapport à une section choisie dudit sujet et en vis-à-vis de N détecteurs (6) précités, les moyens formant horloge étant utilisès, en mode cinedensigraphie, à la place des signaux qui, dans le mode tomodenssitométrie, indiquent la position angulaire du dispositif de mesure.

2. Appareil selon la revendication 1, caractérisé en ce que la fréquence des signaux d'horloge (9) est constante.

3. Appareil selon la revendication 1, caractérisé en ce que la fréquence des signaux d'horloge (9) est réglable par l'opérateur.

4. Appareil selon la revendication 1 ou 2, caractérisé en ce que ladite horloge (9) est connectée à des moyens de mesure d'un phénomène biologique, pour être pilotée par eux.

5. Procédé d'utilisation de l'appareil selon l'une des revendications précédentes pour obtenir, conformément à la technique de la cinédensigraphie, des informations caractéristiques de l'évolution dans le temps de la densité de parties d'un sujet irradié par des rayons X délivrés par ledit appareil, caractérisé en ce qu'il consiste:

- à maintenir ladite source de rayons X dans une position donnée par rapport à une section choisie dudit sujet et en vis-à-vis de N détecteurs précités;

- à soumettre ladite section aux rayons X délivrés par la source;

- à commander l'actionnement desdits moyens de traitement et de memorisation au rythme des moyens formant horloge (9), specifique au mode opératoire de cinédensigraphie; et

- à visualiser des informations disponibles à la sortie desdits moyens de mémorisation et de traitement sans les soumettre à des algorithmes de reconstruction par tomodensitométrie, ces informations étant représentatives de l'amplitude des successions de N signaux résultant de la lecture des N détecteurs en fonction du temps pour un ou plusieurs faisceaux élémentaires donnés et/ou en même temps pour les différents faisceaux élémentaires.

6. Procédé selon la revendication 5, caractérisé par le fait que pour sélectionner la meilleure incidence pour l'observation cinédensigraphiue d'un phénomène, on choisit une projection prédéterminée en fonction des indications fournies par une image tomodensitomètrique obtenue préalablement de la section transversale, en tournant le dispositif de mesure dans la direction angulaire ainsi déterminée.

7. Procede selon la revendication 6, utilisant un tomodensitomètre à détecteurs en couronne fixe, caractérisé par le fait que pour sélectionner ladite direction angulaire, on tourne la seule source de rayons X dans la direction voulue.

8. Procédé selon l'une des revendications 5 à 7, caractérisé par le fait que l'on fait plusieurs mesures de cinédensigraphie pour différentes positions relatives du statif sur lequel est fixé le dispositif de mesure, le long de l'axe longitudinal du sujet.


**Patentansprüche**

1. Gerät mit einer Tomographievorrichtung vom Typ, welche insbesondere eine Vorrichtung zur Messung der Absorption von Röntgenstrahlen in einem Objekt umfaßt, wobei diese Meßvorrichtung (4) eine rotierende Röntgenquelle enthält, welche ein Bündel in Form eines flachen Fächers aussendet, und wenigstens N Detektoren aufweist, die der Quelle gegen über angeordnet sind, um N elementare Bündel innerhalb die ses Bündels auszuwählen, wobei N eine Zahl größer als oder gleich eins ist, Verarbeitungs- und Speichermittel (10) umfaßt, welche so geschaltet sind, daß sie die von den N-Detektoren abgegebenen Signale empfangen, und Sichtanzeigemittel (11) aufweist, wobei der Ausgang der genannten Verarbeitungs- und Speichermittel an die genannten Sichtanzeigemittel (11) angeschlossen ist, dadurch gekennzeichnet, daß es ferner Mittel (9) zur Steuerung der Aktivierung der genannten Verarbeitungs- und Speichermittel umfaßt, welche einen Taktgeber bilden und speziell in Funktion gesetzt werden, um in den genannten Speichermitteln Kinematographie-Informationen zu speichern, wenn die genannte Röntgenquelle festgehalten wird und in einer gegebenen Stellung bezüglich eines ausgewählten Abschnittes des Objektes gegenüber den vorgenannten N Detektoren (6) aussendet, wobei die einen Taktgeber bildenden Mittel im Kinematographie-Betrieb anstelle der Markiermittel (8) verwendet werden, die im Tomographiebetrieb die Winkelstellung der Meßvorrichtung angeben.

2. Gerät nach Anspruch 1, dadurch gekennzeichnet, daß die Frequenz der Taktsignale (9) konstant ist.

3. Gerät nach Anspruch 1, dadurch gekennzeichnet, daß die Frequenz der Taktsignale (9) durch den Bediener einstellbar ist.

4. Gerät nach Anspruch 1 oder 2, dadurch gekennzeichnet, daß der genannte Taktgeber (9) an Mittel zur Messung eines biologischen Phänomens angeschlossen ist, um durch diese gesteuert zu werden.

5. Verfahren zur Anwendung des Gerätes nach einem der vorstehenden Ansprüche, um entsprechend der Kinematographietechnik Informationen zu erhalten, welche charakteristisch für die zeitliche Entwicklung der Dichte von Teilen eines Objektes sind, das durch von dem Gerät abgegebene Röntgenstrahlen bestrahlt wird, dadurch gekennzeichnet, daß es darin besteht, daß:

- die genannte Röntgenquelle in einer gegebenen Stellung bezüglich eines ausgewählten Abschnitts des Objektes und gegenüber den N vorgenannten Detektoren gehalten wird;

- der genannte Abschnitt den von der Quelle abgegebenen Röntgenstrahlen ausgesetzt wird;

- die Aktivierung der genannten Verarbeitungs- und Speichermittel im Rhythmus der den Taktgeber (9) bildenden Mittel, die für die Kinematographie-Betriebsart spezifisch sind, gesteuert wird; und

- die am Ausgang der genannten Verarbeitungs- und Speichermittel verfügbaren Informationen sichtbar angezeigt werden, ohne daß sie Algorithmen für die Rekonstruktion durch Tomographie unterworfen werden, wobei diese Informationen repräsentativ für die Amplitude der Folgen von N Signalen sind, die sich durch das Auslesen der N Detektoren in Abhängigkeit von der Zeit für eines oder mehrere gegebene Elementarbündel und/oder gleichzeitig für die verschiedenen Elementarbündel ergeben.

6. Verfahren nach Anspruch 5, dadurch gekennzeichnet, daß zum Auswählen des besten Einfallswinkels zur kinematographischen Beobachtung eines Phänomens eine Projektion gewählt wird, die im voraus bestimmt wurde in Abhängigkeit von Angaben, welche durch ein Tomographiebild geliefert werden, das zuvor vom Querschnitt erhalten wurde, in dem die Meßvorrichtung in der so bestimmten Winkelrichtung gedreht wird.

7. Verfahren nach Anspruch 6, bei Verwendung eines Tomographiegerätes mit auf einem festen Kranz angeordne ten Detektoren, dadurch gekennzeichnet, daß zum Auswählen der genannten Winkelrichtung allein die Röntgenquelle in der gewünschten Richtung gedreht wird.

8. Verfahren nach einem der Ansprüche 5 bis 7, dadurch gekennzeichnet, daß mehrere Kinematographiemessungen für verschiedene Relativstellungen zu dem Stativ, auf welchem die Meßvorrichtung befestigt ist, entlang der Längsachse des Objektes durchgeführt werden.

## Claims

1. Apparatus comprising a tomographic device of the type comprising, in particular, a device for the measurement of the X-ray absorption in a subject, this measuring device (4) including a rotary X-ray source emitting a beam in the form of a flat fan, and at least N detectors located in facing relationship to said source to select N elementary beams within said beam, N being a number equal to or exceeding one, processing and storing means (10) connected to receive the signals supplied by the N detectors, and visualization means (11), the output of said processing and storing means being connected to said visualization means (11), characterized in that it further comprises means (9) for the activation of said processing and storing means, forming a clock which is specifically used to store within said storing means cinematographic information when said X-ray source is immobilized and emits in a given position with respect to a selected section of said subject, and in facing relationship to the N abovemmentioned detectors (8), the means forming a clock being used, in the cinematographic mode, in place of the marking means (8) which, in the tomographic mode indicate the angular position of the measuring device.

2. Apparatus according to claim 1, characterized in that the frequency of the clock signals (9) is constant.

3. Apparatus according to claim 1, characterized in that the frequency of the clock signals (9) is adjustable by the operator.

4. Apparatus according to claims 1 or 2, characterized in that said clock (9) is connected to means for the measurement of a biologic phenomenon to be controlled thereby.

5. Method of using the apparatus according to any of the preceding claims for obtaining, in accordance with the cinematographic technique, information which is characteristic of the evolution in time of the density of portions of a subject exposed to the X-rays supplied by said apparatus, characterized in that it consists in:

- maintaining said X-ray source in a given position with respect to a selected section of said subject and in facing relationship to the N above-mentioned detectors;

- exposing said section to the X-rays supplied by the source;

- controlling the activation of said processing and storing means in the rhythm of the means (9) forming a clock and specific for the cinematographic mode; and

- visualizing the information available at the output of said processing and storing means without subjecting them to tomographic reconstruction algorithms, this information being representative of the amplitude of successions of N signals resulting from the reading of N detectors in function of time for one or a plurality of given elementary beams and/or simultaneously for the different elementary

**0 022 722**

beams.

6. Method according to claim 5, characterized by the fact that, for selecting the best incidence of cinematographic observation of a phenomenon, a projection is selected which is predetermined in function of indications supplied by a tomographic image previously obtained from the cross section, by turning the measuring device in the thus determined angular direction.

7. Method according to claim 6, using a tomographic device with detectors arranged on a stationary crown, characterized by the fact that only the X-ray source is turned in the desired direction to select said angular direction.

8. Method according to any of claims 5 to 7, characterized by the fact that a plurality of cinematographic measurements are taken for different relative positions of the mount whereon the measuring device is fixed along the longitudinal axis of the subject.

**0 022 722**

# FIG_1

angularcoders

clock

progressing and stokage means

# FIG_2

$t_1$   $P_1$

$t_2$   $P_2$

$P_3$

$t_n$   $P_n$

1

FIG_3

31

32

FIG_4

41

43

42 ———— t

43 ———— t

FIG_5a

51 52

1

2

x

y

z

O

FIG_5b

Pm

1

P1

Pi

P2

z

O

y

y